# EUROPEAN PATENT APPLICATION

(11) **EP 4 837 503 A1**
(43) Date of publication of application: **11.11.2026**
(21) Application number: 26177326.1
(22) Date of filing: 07.05.2026
(51) Int. Cl.: A61B 5/0536, A61B 5/00, A61B 5/08, A61B 5/085

(54) **DATA PROCESSING METHOD BASED ON ELECTRICAL IMPEDANCE, MEDICAL SYSTEM, AND VENTILATION DEVICE**

(30) Priority: 07.05.2025 CN 202510585707
(71) Applicant: Shenzhen Mindray Bio-Medical Electronics Co., Ltd., Shenzhen, Guangdong 518057 (CN); Zhongda Hospital Southeast University, Nanjing, Jiangsu 210009 (CN)
(72) Inventor: YUAN, Xueyan, Shenzhen, 518057 (CN); YAO, Pulin, Shenzhen, 518057 (CN); LIU, Ling, Shenzhen, 518057 (CN); LIU, Jinglei, Shenzhen, 518057 (CN); PANG, Fanghao, Shenzhen, 518057 (CN)
(74) Representative: KIPA AB

(57) **Abstract**

Disclosed are a data processing method based on electrical impedance, a medical system, and a ventilation device. The method includes displaying an opening pressure view of a ventilation subject, wherein the opening pressure view is generated according to ventilation pressure data and electrical impedance data acquired while providing mechanical ventilation to the ventilation subject; determining a measurement region in the opening pressure view, wherein the measurement region includes multiple pixels, wherein each pixel corresponds to one group of opening pressure data; performing statistics on the opening pressure data corresponding to the multiple pixels in the measurement region, so as to obtain a statistical result; and displaying the statistical result. This disclosure presents spatial heterogeneity and time heterogeneity of different pulmonary regions through the opening pressure view, and presents a comprehensive level of opening pressure in a measurement region through a statistical result of opening pressure data within the measurement region.

## Description

### CROSS-REFERENCE TO RELATED APPLICATIONS

This application claims priority to a Chinese patent application filed to CPO on May 07, 2025, with an application number 202510585707.1, and titled "DATA PROCESSING METHOD BASED ON ELECTRICAL IMPEDANCE, MEDICAL SYSTEM, AND VENTILATION DEVICE", the entire contents of which are incorporated by reference into this disclosure.

### TECHNICAL FIELD

The disclosure relates to a medical field, and more particularly to a data processing method based on electrical impedance, a medical system, and a ventilation device.

### BACKGROUND

Thoracic Electrical Impedance Tomography (EIT) is a non-invasive, real-time, continuous bedside monitoring imaging technique, mainly used for dynamic monitoring of regional pulmonary ventilation. At present, an application of EIT in a field of medical imaging is mainly focused on an evaluation of intrapulmonary ventilation distribution in patients with spontaneous respiration and mechanical ventilation. Recent studies have confirmed that EIT can monitor regional airway opening pressure. However, there is still a lack of methods and tools that directly reflect heterogeneity of regional intrapulmonary airway opening pressure.

### SUMMARY

A series of simplified concepts are introduced into summary of this disclosure, and are further elaborated in specific embodiments of this disclosure. The summary of this disclosure does not attempt to define key and necessary technical characteristics of the claimed technical solution, nor attempt to determine a protection scope of the claimed technical solution.

A first aspect according to an embodiment of this disclosure provides a data processing method based on electrical impedance, including:
displaying an opening pressure view of a ventilation subject, wherein the opening pressure view is generated according to ventilation pressure data that reflect a ventilation state and according to electrical impedance data of the ventilation subject; wherein the ventilation pressure data and the electrical impedance data are acquired, while providing mechanical ventilation to the ventilation subject;
determining a measurement region in the opening pressure view, wherein the measurement region includes multiple pixels, wherein each pixel corresponds to one group of opening pressure data;
performing statistics on the opening pressure data, which data correspond to the multiple pixels in the measurement region, so as to obtain a statistical result; and
displaying the statistical result.

In an embodiment, the method further includes:
determining regional opening time that corresponds to each sub-region of multiple sub-regions of lungs of the ventilation subject, based on a change of the electrical impedance data, which data correspond to said each sub-region, over time;
determining the opening pressure data that correspond to said each sub-region, according to the regional opening time that corresponds to said each sub-region, and according to the ventilation pressure data that correspond to the regional opening time; and
generating the opening pressure view based on the opening pressure data that correspond to the multiple sub-regions, wherein each pixel in the opening pressure view corresponds to one sub-region of the multiple sub-regions.

In an embodiment, the statistical result includes at least one of: a mean value, a standard deviation, a maximum value, and a minimum value of the opening pressure data, which data correspond to the multiple pixels in the measurement region.

In an embodiment, determining a measurement region in the opening pressure view, includes:
determining the measurement region based on a user operation received; or
automatically determining the measurement region based on a preset condition.

In an embodiment, automatically determining the measurement region based on a preset condition, includes:
arranging at least one measurement region at a preset position of the opening pressure view; or
dividing the opening pressure view into at least two parts, and determining each part as one measurement region; or
determining the opening pressure view entirely as one measurement region.

In an embodiment, displaying the statistical result, includes:
displaying the statistical result inside the opening pressure view, or
displaying the statistical result outside the opening pressure view.

In an embodiment, the method further includes:
generating ventilation suggestion information based on the statistical result; and
outputting the ventilation suggestion information.

In an embodiment, determining regional opening time that corresponds to each sub-region of multiple sub-regions of lungs of the ventilation subject, based on a change of the electrical impedance data, which data correspond to said each sub-region, over time, includes:
determining the regional opening time that corresponds to said each sub-region in a respiratory cycle, according to time for the electrical impedance data that correspond to said each sub-region to reach an inflection point in an inspiratory phase of the respiratory cycle.

In an embodiment, determining regional opening time that corresponds to each sub-region of multiple sub-regions of lungs of the ventilation subject, based on a change of the electrical impedance data, which data correspond to said each sub-region, over time, includes:
determining the regional opening time that corresponds to said each sub-region in a respiratory cycle, according to time for the electrical impedance data that correspond to said each sub-region to reach a preset threshold in an inspiratory phase of the respiratory cycle.

In an embodiment, the method further includes:
generating an electrical impedance tomography according to the electrical impedance data, wherein the electrical impedance tomography includes multiple pixels, wherein each pixel corresponds to one group of the electrical impedance data; and
displaying the electrical impedance tomography and the opening pressure view in a superimposed manner or in a side-by-side manner.

In an embodiment, providing mechanical ventilation to the ventilation subject, includes:
providing a low-flow lung recruitment maneuver to the ventilation subject.

In an embodiment, the method further includes:
displaying the ventilation pressure data.

In an embodiment, the ventilation pressure data include at least one of: airway pressure data, esophageal pressure data, transpulmonary pressure data, and intrapulmonary pressure data.

In another aspect, an embodiment of this disclosure provides a medical system, including a ventilation device, an electrical impedance apparatus and a processor; wherein the ventilation device includes a sensor and a breathing circuit, wherein the breathing circuit is configured to deliver a gas to a ventilation subject for mechanical ventilation; the sensor is configured to acquire ventilation pressure data that reflect a ventilation state, while providing the mechanical ventilation to the ventilation subject;
the electrical impedance apparatus includes an unit for acquiring electrical impedance data and an unit for processing electrical impedance data; wherein the unit for acquiring electrical impedance data is configured to acquire electrical impedance signals, and the unit for processing electrical impedance data is configured to process the electrical impedance signals, so as to obtain the electrical impedance data;
the processor is configured to implement the data processing method based on electrical impedance as described above.

In yet another aspect, an embodiment of this disclosure provides a ventilation device, including:
a breathing circuit, which is configured to deliver a gas to a ventilation subject for mechanical ventilation;
a sensor, which is configured to acquire ventilation pressure data that reflect a ventilation state, while providing the mechanical ventilation to the ventilation subject;
a communication module, which is in communicative connection with an electrical impedance apparatus, wherein the electrical impedance apparatus is configured to acquire electrical impedance data, while providing the mechanical ventilation to the ventilation subject;
a processor, which is configured to implement the data processing method based on electrical impedance as described above.

The data processing method based on electrical impedance, the medical system, and the ventilation device according to embodiments of this disclosure can present spatial heterogeneity and time heterogeneity of different pulmonary regions during mechanical ventilation through the opening pressure view, and present a comprehensive level of opening pressure in a measurement region through a statistical result of multiple opening pressure data within the measurement region.

### BRIEF DESCRIPTION OF THE DRAWINGS

To illustrate the technical solutions in the embodiments of this disclosure more clearly, the following briefly introduces the accompanying drawings used in the description of the embodiments. Obviously, the accompanying drawings described below are merely some embodiments of this disclosure. For those of ordinary skill in the art, other drawings can be obtained from these drawings without creative effort.

In the accompanying drawings:
FIG. 1 shows a schematic flow chart of a data processing method based on electrical impedance according to an embodiment of this disclosure.
FIG. 2 shows a schematic diagram of determining regional opening time based on electrical impedance data according to an embodiment of this disclosure.
FIG. 3 shows a lung opening pressure view according to an embodiment of this disclosure.
FIG. 4 shows a schematic block diagram of a medical system according to an embodiment of this disclosure.
FIG. 5 shows a schematic block diagram of a ventilation device according to an embodiment of this disclosure.

### DESCRIPTION OF THE PREFERRED EMBODIMENTS

In order to make the purpose, technical solution, and advantages of this disclosure more obvious, the following refers to the attached drawings to describe in detail the exemplary embodiments according to this disclosure. Obviously, the described embodiments are only some of the embodiments of this disclosure, not all of them. It should be understood that this disclosure is not limited by the embodiments described here. Based on the embodiments described in this disclosure, all other embodiments obtained by those skilled in the art without creative work, fall within the protection scope of this disclosure.

In the following description, a large number of specific details are provided to provide a more thorough understanding of this disclosure. However, it is evident to those skilled in the art that this disclosure can be implemented without requiring one or more of these details. In other examples, in order to avoid confusion with this disclosure, some well-known technical characteristics in this field are not described.

It should be understood that this disclosure can be implemented in different forms and should not be limited to the embodiments proposed here. On the contrary, providing these embodiments make the disclosure thorough and complete, and fully convey the scope of this disclosure to those skilled in the art.

The purpose of the terminology used here is only to describe specific embodiments and is not a limitation of this disclosure. When used here, the singular forms of "an", "a", and "the/said" are also intended to include the plural form, unless the context clearly represents another way. It should also be understood that the terms, such as "include", "and/or", "consist of", when used in this disclosure, determine the presence of the characteristics, integers, steps, operations, components, and/or elements, but do not exclude the presence or addition of one or more other characteristics, integers, steps, operations, components, and/or elements. When used here, the term "and/or" includes any and all combinations of related listed items.

In patients with acute respiratory failure undergoing mechanical ventilation, monitoring of airway pressure is essential. Plateau pressure and driving pressure are key targets for ventilation settings and have been proven to be closely related to patient prognosis. However, a static airway pressure cannot reflect an alveolar pressure. Airway closure represents a mismatch between an airway pressure and an alveolar pressure. Airway closure is a physiological phenomenon in which a distal airway becomes obstructed, when an airway pressure falls below a given threshold during expiration. In this situation, alveoli may remain inflated, even though no gas exchange occurs between the alveoli and the distal airway. Alveoli can be re-inflated only when the closed airway reopens. Therefore, evaluating whether an airway closure exists and evaluating an airway opening pressure may represent an important approach to guide setting of ventilator parameters. Previous studies have confirmed that a P-V curve is a reliable method for assessing an airway closure. Nevertheless, the P-V curve cannot reflect a regional airway closure or an opening pressure level. Thus, it fails to provide a precise opening pressure level of regional intrapulmonary airways/alveoli.

Thoracic Electrical Impedance Tomography (EIT) is a non-invasive, real-time, continuous bedside monitoring imaging technique, mainly used for dynamic monitoring of regional pulmonary ventilation, which may involve alveoli or airway monitoring. At present, an application of EIT in a field of medical imaging is mainly focused on an evaluation of intrapulmonary ventilation distribution in patients with spontaneous respiration and mechanical ventilation. EIT is capable of monitoring a regional airway opening pressure, and of monitoring an airway opening pressure level of a single lung, by assessing a pressure-impedance curve within the single lung under mechanical ventilation, the airway opening pressure level of the single lung can be monitored. Airway opening pressure levels also differ significantly among different intrapulmonary regions, which may be associated with a severity of disease in mechanically ventilated patients.

In order to fully understand this disclosure, a detailed structure is provided in the following description to explain the technical solution proposed in this disclosure. The optional embodiments of this disclosure are described in detail below, however, in addition to these detailed description, this disclosure may also have other embodiments.

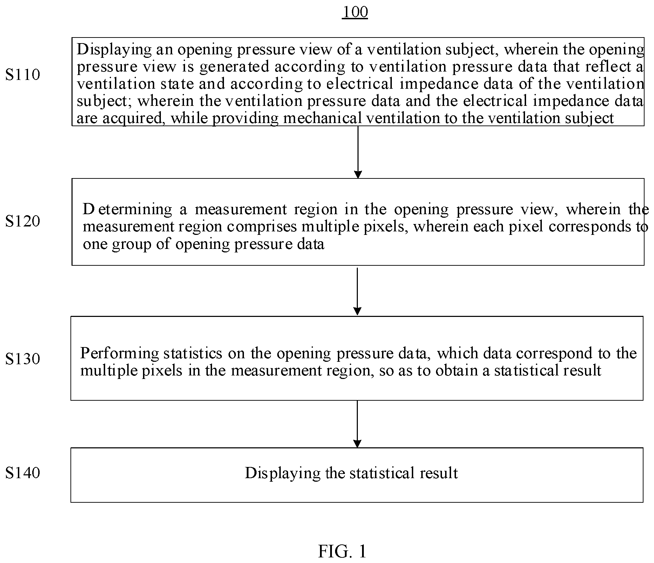

Hereinafter, a data processing method based on electrical impedance according to embodiments of this disclosure will first be described with reference to FIG. 1. FIG. 1 is a schematic flow chart of a data processing method 100 based on electrical impedance in an embodiment of this disclosure, which specifically includes following steps.

Step S110, display an opening pressure view of a ventilation subject, wherein the opening pressure view is generated according to ventilation pressure data that reflect a ventilation state and according to electrical impedance data of the ventilation subject; wherein the ventilation pressure data and the electrical impedance data are acquired, while providing mechanical ventilation to the ventilation subject.

Step S120, determine a measurement region in the opening pressure view, wherein the measurement region includes multiple pixels, wherein each pixel corresponds to one group of opening pressure data.

Step S130, perform statistics on the opening pressure data, which data correspond to the multiple pixels in the measurement region, so as to obtain a statistical result.

Step S140, display the statistical result.

The data processing method 100 based on electrical impedance according to embodiments of this disclosure may be implemented in a ventilation device. The ventilation device is configured to deliver a gas to a ventilation subject to perform ventilation. Exemplarily, the ventilation device is in communicative connection with an electrical impedance apparatus, so as to control the electrical impedance apparatus to acquire electrical impedance data during ventilation, and so as to obtain, from the electrical impedance apparatus, the electrical impedance data acquired by the electrical impedance apparatus. Alternatively, the ventilation device is integrated with the electrical impedance apparatus, and the electrical impedance apparatus may serve as a functional module of the ventilation device. The data processing method 100 based on electrical impedance according to embodiments of this disclosure may also be implemented in the electrical impedance apparatus, or a third-party device that is in communicative connection with the ventilation device and the electrical impedance apparatus.

In Step S110, it is possible to determine regional opening time that corresponds to multiple sub-regions of lungs of the ventilation subject according to a change of the electrical impedance data over time; determine the opening pressure data that correspond to each sub-region of the multiple sub-regions according to ventilation pressure data, which data correspond to the regional opening time that corresponds to said each sub-region; and generate the opening pressure view according to the opening pressure data that correspond to the multiple sub-regions. Alternatively, an opening pressure view that is generated by another device may be acquired and displayed. The reason why the electrical impedance data can be used to determine the regional opening time is that a magnitude of electrical impedance is positively correlated with a volume of gas in a region. For example, in an inspiratory phase, a gas enters alveoli and an electrical impedance increases gradually; in an expiratory phase, the gas in the alveoli is expelled and the electrical impedance decreases gradually. Therefore, time, when alveoli are opened and effective ventilation is performed, namely the regional opening time, can be determined according to the electrical impedance data. The ventilation pressure data that correspond to the regional opening time is the opening pressure data. The opening pressure data can reflect a difficulty degree of regional opening; wherein the greater the opening pressure data, the more difficult the regional opening. Displaying the opening pressure data that correspond to different sub-regions can reflect a time heterogeneity of lung ventilation.

Wherein, the electrical impedance data and the ventilation pressure data are acquired, while a ventilation device providing mechanical ventilation to the ventilation subject, wherein the ventilation device providing mechanical ventilation to the ventilation subject refers to a process in which the ventilation device delivers a gas to the ventilation subject via a breathing circuit. Exemplarily, providing mechanical ventilation to the ventilation subject includes providing a low-flow lung recruitment maneuver to the ventilation subject, and the low-flow lung recruitment maneuver refers to that a ventilation flow rate is less than or equal to 10 L/min.

Exemplarily, the ventilation pressure data include, but is not limited to, at least one of: airway pressure data, esophageal pressure data, transpulmonary pressure data, and intrapulmonary pressure data. Wherein, the intrapulmonary pressure (Plung) changes relatively obviously with the regional opening time, and can better reflect the time heterogeneity during ventilation. The intrapulmonary pressure (Plung) can be calculated based on an airway pressure (Paw) according to a respiratory mechanics equation. The ventilation pressure data acquired during low-flow lung recruitment is closer to an intrapulmonary pressure. The ventilation pressure data can be displayed on the same screen as the opening pressure view generated subsequently.

During ventilation, in addition to acquiring the above ventilation pressure data that reflect a ventilation state, electrical impedance data of the ventilation subject is also acquired by an electrical impedance apparatus. The magnitude of electrical impedance is affected by a gas content inside lungs and can reflect a pulmonary ventilation condition. The electrical impedance data and the ventilation pressure data are acquired synchronously, and time alignment can be performed on the electrical impedance data and the ventilation pressure data according to a time stamp.

Exemplarily, the electrical impedance apparatus includes multiple electrodes (e.g., 16 electrodes). During electrical impedance tomography, a current is first applied to a pair of electrodes, so as to enable said pair of electrodes to emit a current, the remaining electrodes detect a voltage change on the human body surface immediately, so as to obtain a surface potential difference. Subsequently, a second pair of electrodes emits a current, the remaining electrodes detect a voltage change again, and the process is repeated continuously, finally obtaining a conductivity distribution of tissue. Multiple sectional views can be obtained in each current cycle, and electrical impedance data that reflect changes in the pulmonary electrical impedance can be obtained by combining all the sectional views. An electrical impedance tomography can be generated according to the electrical impedance data, wherein the electrical impedance tomography includes multiple pixels, each pixel corresponds to one group of electrical impedance data.

After acquiring the electrical impedance data, preprocessing may also be performed on the electrical impedance data, for example, de-noising processing, such as signal filtering processing and image filtering processing, can be performed on the electrical impedance data. Exemplarily, contact impedance between the electrodes and the patient may also be acquired, when the electrical impedance apparatus collects the electrical impedance data, and whether the contact impedance exceeds a preset threshold of contact impedance can be determined. If the contact impedance exceeds the preset threshold of contact impedance, it indicates a poor contact between the electrodes and skin of the patient, and the acquired electrical impedance data should be discarded.

Next, regional opening time that corresponds to each sub-region of lungs is determined according to a change of the electrical impedance data that correspond to each sub-region of the lungs. Since a gravity and an injury degree are inconsistent in different pulmonary regions, the difficulty degree of regional opening is different in different regions, and thus differences exist in the regional opening time. Considering that the magnitude of electrical impedance is related to the gas content in the region, the regional opening time that corresponds to different sub-regions can be determined according to the change of the electrical impedance data. After determining the regional opening time that corresponds to the multiple sub-regions, opening pressure data that correspond to said each sub-region is determined, according to the regional opening time that corresponds to each sub-region and the ventilation pressure data that correspond to said regional opening time. The larger the value of the opening pressure data, the more difficult the regional opening of a corresponding sub-region.

In some embodiments, each sub-region may correspond to one pixel in the electrical impedance tomography. In this case, the regional opening time that corresponds to a pixel can be obtained based on a change of the electrical impedance data, which data correspond to said pixel, over time. In other embodiments, each sub-region may also correspond to a pixel set that is composed of multiple adjacent pixels in the electrical impedance tomography. In this case, the regional opening time that corresponds to a pixel set can be determined based on a statistical value of the electrical impedance data that correspond to the multiple pixels in said pixel set; wherein the statistical value of the electrical impedance data include a mean value, a maximum value, a minimum value, etc., of the electrical impedance data that correspond to the multiple pixels. In some embodiments, each pixel in a sub-region has a same regional opening state, or in other words, multiple pixels having a same regional opening state are counted as one sub-region. When determining the regional opening time that corresponds to the multiple sub-regions, the regional opening time of the multiple sub-regions in a same respiratory cycle can be determined.

Exemplarily, since a change amount of electrical impedance data is positively correlated with a gas volume that enters into and leaves out of a region (e.g., alveoli), when the region opens, gas begins to enter into the alveoli, and the electrical impedance data will increase significantly. Therefore, regional opening time of each sub-region in at least one respiratory cycle can be determined according to time for the electrical impedance data that correspond to said each sub-region to reach an inflection point in an inspiratory phase of said at least one respiratory cycle. Alternatively, the regional opening time of each sub-region in the respiratory cycle is determined according to time for the electrical impedance data that correspond to said each sub-region to reach a preset threshold in an inspiratory phase of said at least one respiratory cycle. After determining the regional opening time of each sub-region, an opening pressure of said each sub-region can be obtained according to ventilation pressure data that correspond to the regional opening time of said sub-region. For example, an expression for an opening pressure that corresponds to pixel N is: ${Popen}_{pixelN} = P \left({t}_{open}\right)$ (Formula 1) wherein, *tₒₚₑₙ* is regional opening time that corresponds to pixel N.

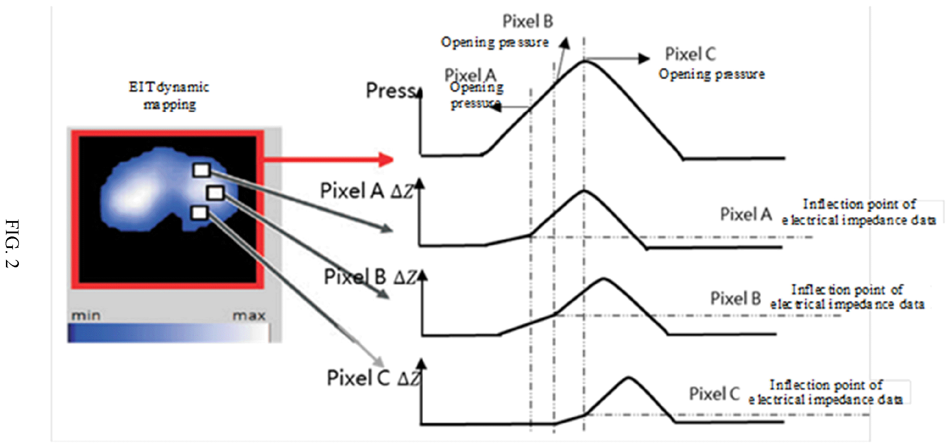

Referring to FIG. 2, a Local Electrical Impedance Tomogram corresponding to one or more pixels can be generated according to a change of electrical impedance data, which data correspond to the one or more pixels, over time. A curve for a change of electrical impedance data, which data correspond to all pixels, over time, can be referred to as a Global Electrical Impedance Tomogram. FIG. 2 sequentially shows, from top to bottom, a change curve of ventilation pressure data, and the Local Electrical Impedance Tomograms of pixel A, pixel B and pixel C. When the electrical impedance data of a certain pixel reaches an inflection point and an increase rate becomes greater, it indicates that alveoli and/or airway corresponding to the pixel has/have been opened. The respective regional opening time of pixel A, pixel B and pixel C are delayed in sequence, indicating that respective regional opening difficulties of pixel A, pixel B and pixel C increase in sequence.

In some embodiments, an electrical impedance threshold corresponding to each sub-region can be determined according to a peak value reached by the electrical impedance data that correspond to said each sub-region in a respiratory cycle. For example, the electrical impedance threshold can be 10% of a peak value of electrical impedance in one respiratory cycle. In this example, an expression of an electrical impedance threshold corresponding to pixel N is: $Δ {Z}_{pixelN} \left({t}_{open}\right) = 10 %Max Δ {Z}_{pixelN}$ (Formula 2).

In Formula 2, *Max*Δ*Z_{pixelN}* is a maximum value of electrical impedance data of pixel N in an inspiratory phase.

Optionally, the time for the electrical impedance data to reach the inflection point can also be determined by means of curve fitting. For example, the inflection point can be determined by means of Sigmoid curve linear fitting: ${\hat{Z}}_{pixelN} = f \left(t\right) = a + \frac{b}{1 + {e}^{- \left(t-c\right) / d}}$ (Formula 3) ${t}_{open} = c - 2 d$ (Formula 4).

In Formula 3, the electrical impedance curve is fitted into a function f(t) to obtain values of several parameters a, b, c, d, and then the regional opening time *tₒₚₑₙ* is determined according to Formula 4.

Alternatively, the inflection point can be determined by means of piecewise linear fitting: ${\hat{Z}}_{pixelN} = f \left(t\right) = \left\{\begin{matrix}{a}_{1} t + {b}_{1} & 0 \leq t < {t}_{open} \\ {a}_{2} t + {b}_{2} & t \geq {t}_{open}\end{matrix}\right.$ (Formula 5).

In Formula 5, *a*₁*, b*₁ are slope and intercept of the fitting line before the inflection point, and *a*₂, *b*₂ are slope and intercept of the fitting line after the inflection point.

In addition, the inflection point can also be calculated through a curve of electrical impedance data varying with pressure (P-ΔZ curve).

After obtaining opening pressure data that correspond to multiple sub-regions, an opening pressure view can be generated according to the opening pressure data of the multiple sub-regions. Each pixel in the opening pressure view corresponds to one sub-region, or each pixel in the opening pressure view corresponds to multiple sub-regions. Specifically, magnitudes of the opening pressure data of different sub-regions can be mapped to image parameters of corresponding regions in the opening pressure view. For example, a color value of each sub-region can be determined according to the magnitude of the opening pressure data of said each sub-region, so as to obtain an opening pressure view for presenting regional opening difficulties of different sub-regions, and simultaneously presenting time heterogeneity and spatial heterogeneity of lung ventilation. In the opening pressure view, the more obvious the color difference between different regions, the more prominent the time heterogeneity of the lung ventilation and the greater the respiratory mechanics difference between different regions. Conversely, if the color difference between different regions in the opening pressure view is small and the color of the entire opening pressure view is uniform, it indicates that the time heterogeneity of the lung ventilation is not obvious.

When generating the opening pressure view, the opening pressure data may be determined according to the regional opening time that corresponds to each sub-region in one respiratory cycle, so as to generate the opening pressure view. Alternatively, the opening pressure data that respectively correspond to each sub-region in multiple respiratory cycles may be obtained according to the regional opening time that corresponds to said each sub-region in said multiple respiratory cycles; and a statistical operation, such as averaging, may be performed on the opening pressure data that respectively correspond to each sub-region in multiple respiratory cycles, so as to obtain a statistical value of the opening pressure data, and the opening pressure view may be generated according to the statistical value of the opening pressure data that correspond to the multiple sub-regions, so as to reduce possible errors.

Further, when generating the opening pressure view, a target range of the opening pressure data may also be acquired, and pixels, which correspond to sub-regions that correspond to opening pressure data outside of the target range, may be displayed differently in the opening pressure view, so as to avoid misleading the user by abnormal opening pressure data. For example, pixels, which correspond to sub-regions that correspond to opening pressure data within the target range, may be displayed in colors; and pixels, which correspond to sub-regions that correspond to opening pressure data outside the target range, may be displayed in gray or white.

In some embodiments, the electrical impedance tomography and the opening pressure view are displayed in a side-by-side manner or a superimposed manner. Wherein, displaying in a side-by-side manner displaying the opening pressure view and the electrical impedance tomography respectively, so as to facilitate the user to view the opening pressure view and the electrical impedance tomography, respectively. In such a way, on the one hand, the user learns the spatial heterogeneity in the ventilation process through the electrical impedance tomography, and on the other hand, learns the time heterogeneity in the ventilation process through the opening pressure view. Displaying in a superimposed manner may include superimposing the opening pressure view onto the electrical impedance tomography with a certain transparency, wherein superimposing the opening pressure view and the electrical impedance tomography facilitates comparative analysis thereof.

The opening pressure view can qualitatively present the regional opening difficulty of different sub-regions, but cannot directly reflect the heterogeneity of opening pressure levels quantitatively. Aiming at this problem, in the embodiment of this disclosure, a measurement region is determined in the opening pressure view, and statistics are performed on the opening pressure data that correspond to multiple pixels in the measurement region to obtain a statistical result. The statistical result can directly reflect a comprehensive level of opening pressure quantitatively, and provide clinical guidance for mechanical ventilation and lung recruitment.

Wherein, the measurement region may be manually determined by the user, that is, the measurement region may be determined based on a user operation received. The user may set a measurement region in a region of interest. For example, if a certain region of the opening pressure view has an obvious color difference, the user may set a measurement region in the region to view a statistical value of the opening pressure data in the region. Alternatively, the user may set multiple measurement regions in different regions respectively, so as to compare opening pressure differences of different regions.

Exemplarily, a measurement function key may be displayed in a display interface, and a function for selecting a measurement region is activated in response to a selection operation on the measurement function key. At this time, the user may select a measurement region in the opening pressure view. Exemplarily, the measurement region selected by the user may have a preset shape, such as a circle or a square, the user may also flexibly draw a measurement region of any size and shape. After the user determines a currently selected measurement region, the processor automatically performs statistics on the opening pressure data that correspond to the multiple pixels in the measurement region, and displays the statistical result. Exemplarily, the user may be allowed to select multiple measurement regions on the opening pressure view, and respectively display the statistical result of each measurement region, so as to compare comprehensive levels of opening pressure for different measurement regions.

In another embodiment, the measurement region may also be automatically determined based on a preset condition. When the measurement region is automatically determined, the measurement region may be automatically determined, while displaying the opening pressure view, and the statistical result may be displayed synchronously, or the measurement region may be automatically determined and the statistical result may be displayed in response to a selection operation of a user on the measurement function key.

In an embodiment, automatically determining the measurement region based on a preset condition may include arranging at least one measurement region at a preset position of the opening pressure view. For example, one measurement region may be arranged at a central position of each pulmonary region. The division manner of pulmonary region may be dividing the opening pressure view into a left pulmonary region and a right pulmonary region, or further dividing the left and right pulmonary regions into upper and lower regions or front and rear regions. The measurement region arranged in each pulmonary region may have a preset size and/or a preset shape. The preset position in the opening pressure view may also be other positions in the opening pressure view that satisfy a preset condition. For example, a region with high heterogeneity in the opening pressure view may be identified and taken as the measurement region, or measurement regions may be arranged respectively in a gravity-dependent region and a non-gravity-dependent region, so as to compare opening pressure differences there between.

Alternatively, automatically determining the measurement region based on a preset condition may include dividing the opening pressure view into at least two parts, and determining each part as one measurement region. For example, the opening pressure view may be divided into a left pulmonary region view and a right pulmonary region view, statistics are performed on the opening pressure data that correspond to all pixels in the left pulmonary region view to obtain a statistical result corresponding to the left pulmonary region view, and statistics are performed on the opening pressure data that correspond to all pixels in the right pulmonary region view to obtain a statistical result corresponding to the right pulmonary region view. The user may compare comprehensive levels of opening pressure of different pulmonary regions.

In some embodiments, the entire opening pressure view may also be determined as one measurement region, that is, statistics are performed on the opening pressure data that correspond to all pixels in the opening pressure view to obtain a global statistical result, which is used for reflecting the whole-lung opening pressure level.

Exemplarily, the statistical result of the opening pressure data includes at least one of: a mean value, a standard deviation, a maximum value, a minimum value, a median value, a percentile value, etc. of the opening pressure data that correspond to the multiple pixels in the measurement region. The mean value of the opening pressure data that correspond to the multiple pixels in the measurement region can reflect an overall opening pressure level of the measurement region; the standard deviation of the opening pressure data that correspond to the multiple pixels in the measurement region can reflect an opening pressure heterogeneity of the measurement region; the maximum value and the minimum value of the opening pressure data that correspond to the multiple pixels in the measurement region can reflect extreme conditions of the opening pressure. Wherein, calculation formulas of the mean value and the standard deviation are as follows: $\overline{x} = \frac{{\sum }_{i = 1}^{n} {x}_{i}}{n}$ (Formula 6) $SD = \sqrt{\frac{{\sum }_{i = 1}^{n} {\left({x}_{i}-\overline{x}\right)}^{2}}{n - 1}}$ (Formula 7)

Wherein, *x̅* represents the mean value of the opening pressure data, SD represents the standard deviation of the opening pressure data, *xᵢ* is the opening pressure data of the i-th pixel, and n is the number of pixels in the measurement region.

Exemplarily, the statistical result may be displayed in the opening pressure view, for example, displayed near the measurement region, so as to reflect the corresponding relationship between the statistical result and the measurement region. Alternatively, the statistical result may also be displayed outside the opening pressure view, so as to avoid blocking the opening pressure view.

After obtaining the statistical result of the measurement region, ventilation suggestion information may also be generated based on the statistical result, and the ventilation suggestion information may be output. For example, a setting of ventilation parameters such as PEEP (Positive End-Expiratory Pressure) may be guided based on the statistical result. Specifically, the setting of ventilation parameters may be guided according to the statistical result of a single measurement region; the setting of ventilation parameters may also be guided according to the statistical results of multiple measurement regions; the setting of ventilation parameters may be guided according to one or more statistical results of the mean value, the standard deviation, the maximum value, the minimum value and the median value. For example, if the standard deviation of the opening pressure data is less than a preset threshold of standard deviation, it indicates that a uniformity of opening pressure is good, the current ventilation pressure has covered an opening demand of most regions, and there is no need to adjust the ventilation pressure; if the standard deviation of the opening pressure data is greater than the preset threshold of standard deviation, it indicates that the opening difficulty varies greatly among different regions, the current ventilation pressure cannot meet the opening demand of all regions, and the ventilation pressure needs to be adjusted, for example, the ventilation pressure may be appropriately increased or other lung recruitment maneuvers may be employed.

Based on the above description, the data processing method 100 based on electrical impedance according to the embodiment of this disclosure can present spatial heterogeneity and time heterogeneity of different pulmonary regions during mechanical ventilation through the opening pressure view, and present the comprehensive level of opening pressure in the measurement region through the statistical result of multiple opening pressure data in the measurement region.

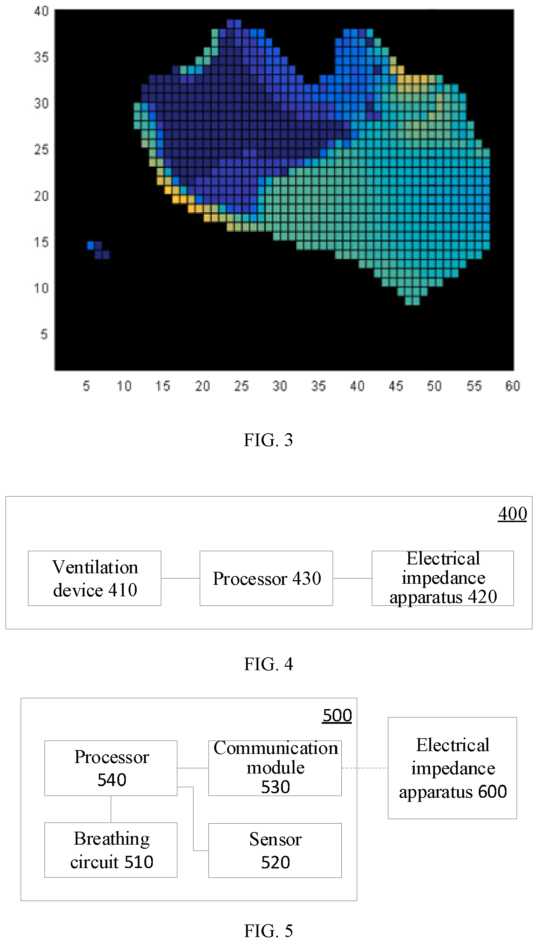

In another aspect, an embodiment of this disclosure provides a medical system. Referring to FIG. 4, the medical system 400 includes a ventilation device 410, an electrical impedance apparatus 420 and a processor 430. The ventilation device 410 includes a sensor and a breathing circuit, wherein the breathing circuit is configured to deliver a gas to a ventilation subject to perform mechanical ventilation. The sensor is configured to acquire ventilation pressure data that reflect a ventilation state, while providing the mechanical ventilation to the ventilation subject. The electrical impedance apparatus 420 includes an unit for acquiring electrical impedance data and an unit for processing electrical impedance data. The unit for acquiring electrical impedance data is configured to acquire electrical impedance signals, and the unit for processing electrical impedance data is configured to process the electrical impedance signals, so as to obtain electrical impedance data. The processor 430 is configured to implement steps of the data processing method 100 based on electrical impedance as described above.

Exemplarily, the ventilation device 410 may be implemented as a medical device having a mechanical ventilation function, such as a ventilator, an anesthesia machine, an oxygen therapy machine, etc. The breathing circuit of the ventilation device 410 is connected with a gas source interface and a ventilation control unit, and is configured to deliver a gas provided by the gas source interface to the ventilation subject under a control of the ventilation control unit. The gas provided by the gas source interface to the ventilation subject may be oxygen, or a mixed gas of air and oxygen.

The electrical impedance apparatus 420 includes an unit for acquiring electrical impedance data and an unit for processing electrical impedance data. The unit for acquiring electrical impedance data is configured to acquire electrical impedance signals, and the unit for processing electrical impedance data is configured to process the electrical impedance signals, so as to obtain electrical impedance data. The unit for acquiring electrical impedance data is connected with multiple electrodes. During electrical impedance tomography, a current is first applied to a pair of electrodes, so as to enable said pair of electrodes to emit a current, the remaining electrodes detect a voltage change on the human body surface immediately, so as to obtain a surface potential difference. Subsequently, a second pair of electrodes emits a current, the remaining electrodes detect a voltage change again, and the process is repeated continuously, finally obtaining a conductivity distribution of tissue. Multiple sectional views can be obtained in one current cycle, and the unit for processing electrical impedance data can obtain electrical impedance data that reflect changes in the pulmonary electrical impedance by combining all the sectional views. In some embodiments, the electrical impedance apparatus 420 may serve as a plug-in module, an external module or a built-in module of the ventilation device 410, i.e., the medical system 400 may be a ventilation device integrated with an electrical impedance tomography module. In other embodiments, the electrical impedance apparatus 420 may be an independent device.

The processor 430 may be a processor of the ventilation device 410, a processor of the electrical impedance apparatus 420, or a processor of a third-party device in communicative connection with the ventilation device 410 and the electrical impedance apparatus 420. The processor 430 may be implemented by software, hardware, firmware or any combination thereof, and may use a circuit, a single or multiple application-specific integrated circuits, a single or multiple general-purpose integrated circuits, a single or multiple microprocessors, a single or multiple programmable logic devices, or any combination of the foregoing circuits and/or devices, or other suitable circuits or devices. Moreover, the processor 430 may control other components in the ventilation device 410, in the electrical impedance apparatus 420 or in the third-party device, so as to perform desired functions.

In an embodiment, the medical system 400 further includes a communication module, which is configured to establish a communication connection between the ventilation device 410 and the processor 430, or between the processor 430 and the electrical impedance apparatus 420. The processor 430 controls the electrical impedance apparatus and/or the ventilation device through the communication connection. For example, when the processor 430 is a processor of the ventilation device 410, the communication module 410 is configured to establish a communication connection between the processor 430 and the electrical impedance apparatus 420, and the processor 430 controls the electrical impedance apparatus 420 through the communication connection.

The medical system 400 according to the embodiment of this disclosure is configured to implement the data processing method 100 based on electrical impedance described above. For specific details of the method, reference may be made to the above content, which will not be repeated herein. The medical system 400 according to the embodiment of this disclosure can present spatial heterogeneity and time heterogeneity of different pulmonary regions during mechanical ventilation through the opening pressure view, and present a comprehensive level of opening pressure in the measurement region through statistical results of multiple opening pressure data in the measurement region.

In another aspect, an embodiment of this disclosure provides a ventilation device, which can implement the data processing method 100 based on electrical impedance described above. Referring to FIG. 5, the ventilation device 500 according to the embodiment of this disclosure includes: a breathing circuit 510 that is configured to deliver a gas to a ventilation subject to perform mechanical ventilation; a sensor 520 that is configured to acquire ventilation pressure data that reflect a ventilation state, while providing the mechanical ventilation to the ventilation subject; a communication module 530 that is in communicative connection with an electrical impedance apparatus, wherein the electrical impedance apparatus is configured to acquire electrical impedance data, when providing the mechanical ventilation to the ventilation subject; and a processor 540 that is configured to implement steps of the data processing method 100 based on electrical impedance.

Wherein, the ventilation device 500 may be implemented as a medical device having a mechanical ventilation function, such as a ventilator, an anesthesia machine, an oxygen therapy machine, etc. The communication connection provided by the communication module 530 may be a wired connection or a wireless connection. The sensor 520 at least includes a pressure sensor that is configured to acquire ventilation pressure data during ventilation. The processor 540 receives electrical impedance data acquired by the electrical impedance apparatus through the communication connection provided by the communication module 530, and implements the steps of the data processing method 100 based on electrical impedance. For specific details of the method, reference may be made to the above content, which will not be repeated herein. The ventilation device 500, according to this disclosure, can present spatial heterogeneity and time heterogeneity of different pulmonary regions during mechanical ventilation through the opening pressure view, and present a comprehensive level of opening pressure in the measurement region through statistical results of multiple opening pressure data in the measurement region.

Although the exemplary embodiments are described here with reference to the accompanying drawings, it should be understood that the aforementioned exemplary embodiments are only illustrative and are not intended to limit the scope of this disclosure. Ordinary technical personnel in this field can make various changes and modifications without deviating from the scope and spirit of this disclosure. All these changes and modifications are intended to be included within the scope of this disclosure as claimed in the attached claims.

Ordinary technical personnel in this field can realize that the units and algorithm steps described in combination with this disclosure can be implemented in electronic hardware, or a combination of computer software and electronic hardware. Whether these functions are executed in hardware or software depends on the specific application and design constraints of the technical solution. Professional technicians may use different methods to implement the described functions for each specific application, but such implementation should not be considered beyond the scope of this disclosure.

In the several embodiments provided in this disclosure, it should be understood that the disclosed devices and methods can be implemented in other ways. For example, the device embodiments described above are only schematic. For example, the division of the units is only a logical functional division, and there may be other division methods in actual implementations. For example, multiple units or components can be combined or integrated to another device, or some characteristics can be ignored or not executed.

The disclosure provided here provides a large number of specific details. However, it can be understood that the embodiments of this disclosure can be practiced without these specific details. In some examples, well-known methods, structures, and techniques are not shown in detail to avoid blurring the understanding of this disclosure.

Similarly, it should be understood that in order to streamline this disclosure and assist in understanding one or more of the various aspects of this disclosure, in the description of exemplary embodiments of this disclosure, the various characteristics of this disclosure are sometimes grouped together into a single embodiment, diagram, or description thereof. However, the method of this disclosure should not be interpreted as reflecting the intention that the claimed protection of this disclosure requires more characteristics than those explicitly recorded in each claim. More precisely, as reflected in the corresponding claims, the inventive point is that the corresponding technical problem can be solved with less than all characteristics of a single disclosed embodiment. Therefore, the claims following the specific implementation method are explicitly incorporated into the specific implementation method, where each claim itself serves as a separate embodiment of this disclosure.

Ordinary technical personnel in this field can understand that, in addition to mutual exclusion between characteristics, any combination can be configured to combine all characteristics disclosed in this disclosure (including accompanying claims, abstract, and drawings), as well as all processes or units of any method or device so disclosed. Unless otherwise explicitly stated, each characteristic disclosed in this disclosure (including accompanying claims, abstract, and accompanying drawings) may be replaced by alternative characteristics that provide the same, equivalent, or similar purpose.

In addition, Ordinary technical personnel in this field can understand that although some embodiments described herein include certain characteristics rather than other characteristics included in other embodiments, the combination of characteristics of different embodiments means that they are within the scope of this disclosure and form different embodiments. For example, in the claims, any one of the claimed embodiments can be used in any combination.

The various component embodiments of this disclosure can be implemented in hardware, software modules running on one or more processors, or a combination thereof. Ordinary technical personnel in this field should understand that a microprocessor or digital signal processor (DSP) can be used in practice to implement some or all of the functions of some modules according to the embodiments of this disclosure. This disclosure can also be implemented as a partial or complete device program (such as a computer program and a computer program product) for executing the method described herein. The program implementing this disclosure can be stored at a computer-readable medium or can have the form of one or more signals. Such signals can be downloaded from internet websites, provided on carrier signals, or in any other form.

It should be noted that the above embodiments illustrate this disclosure rather than limit it, and Ordinary technical personnel in this field can design alternative embodiments without departing from the scope of the attached claims. In the claims, any reference symbol between parentheses should not be constructed as a restriction on the claims. This disclosure can be implemented with the help of hardware consisting of several different components and with the help of appropriately programmed computers. Among the unit claims that list several devices, several of these devices can be specifically embodied through the same hardware item. The use of words, such as first, second, and third, does not indicate any order. These words can be interpreted as names.

The above is only the specific implementation method or explanation of the specific implementation method of this disclosure. The protection scope of this disclosure is not limited to this. Any technical personnel familiar with the technical field can easily think of changes or replacements within the technical scope disclosed in this disclosure, and those changes or replacements should fall into the protection scope of this disclosure. The protection scope of this disclosure shall be based on the protection scope of the claims.

## Claims

1. A data processing method based on electrical impedance, **characterized in that**, comprising:
displaying an opening pressure view of a ventilation subject, wherein the opening pressure view is generated according to ventilation pressure data that reflect a ventilation state and according to electrical impedance data of the ventilation subject; wherein the ventilation pressure data and the electrical impedance data are acquired, while providing mechanical ventilation to the ventilation subject;
determining a measurement region in the opening pressure view, wherein the measurement region comprises multiple pixels, wherein each pixel corresponds to one group of opening pressure data;
performing statistics on the opening pressure data, which data correspond to the multiple pixels in the measurement region, so as to obtain a statistical result; and
displaying the statistical result.

2. The method according to claim 1, **characterized in that**, further comprising:
determining regional opening time that corresponds to each sub-region of multiple sub-regions of lungs of the ventilation subject, based on a change of the electrical impedance data, which data correspond to said each sub-region, over time;
determining the opening pressure data that correspond to said each sub-region, according to the regional opening time that corresponds to said each sub-region, and according to the ventilation pressure data that correspond to the regional opening time; and
generating the opening pressure view based on the opening pressure data that correspond to the multiple sub-regions, wherein each pixel in the opening pressure view corresponds to one sub-region of the multiple sub-regions.

3. The method according to claim 1, **characterized in that**, the statistical result comprises at least one of: a mean value, a standard deviation, a maximum value, and a minimum value of the opening pressure data, which data correspond to the multiple pixels in the measurement region.

4. The method according to claim 1, **characterized in that**, determining a measurement region in the opening pressure view, comprises:
determining the measurement region based on a user operation received; or
automatically determining the measurement region based on a preset condition.

5. The method according to claim 4, **characterized in that**, automatically determining the measurement region based on a preset condition, comprises:
arranging at least one measurement region at a preset position of the opening pressure view; or
dividing the opening pressure view into at least two parts, and determining each part as one measurement region; or
determining the opening pressure view entirely as one measurement region.

6. The method according to claim 1, **characterized in that**, displaying the statistical result, comprises:
displaying the statistical result inside the opening pressure view, or
displaying the statistical result outside the opening pressure view.

7. The method according to claim 1, **characterized in that**, further comprising:
generating ventilation suggestion information based on the statistical result; and
outputting the ventilation suggestion information.

8. The method according to claim 2, **characterized in that**, determining regional opening time that corresponds to each sub-region of multiple sub-regions of lungs of the ventilation subject, based on a change of the electrical impedance data, which data correspond to said each sub-region, over time, comprises:
determining the regional opening time that corresponds to said each sub-region in a respiratory cycle, according to time for the electrical impedance data that correspond to said each sub-region to reach an inflection point in an inspiratory phase of the respiratory cycle.

9. The method according to claim 2, **characterized in that**, determining regional opening time that corresponds to each sub-region of multiple sub-regions of lungs of the ventilation subject, based on a change of the electrical impedance data, which data correspond to said each sub-region, over time, comprises:
determining the regional opening time that corresponds to said each sub-region in a respiratory cycle, according to time for the electrical impedance data that correspond to said each sub-region to reach a preset threshold in an inspiratory phase of the respiratory cycle.

10. The method according to claim 1, **characterized in that**, further comprising:
generating an electrical impedance tomography according to the electrical impedance data, wherein the electrical impedance tomography comprises multiple pixels, wherein each pixel corresponds to one group of the electrical impedance data; and
displaying the electrical impedance tomography and the opening pressure view in a superimposed manner or in a side-by-side manner.

11. The method according to claim 1, **characterized in that**, providing mechanical ventilation to the ventilation subject, comprises:
providing a low-flow lung recruitment maneuver to the ventilation subject.

12. The method according to claim 1, **characterized in that**, further comprising:
displaying the ventilation pressure data.

13. The method according to any one of claims 1~12, **characterized in that**, the ventilation pressure data comprise at least one of: airway pressure data, esophageal pressure data, transpulmonary pressure data, and intrapulmonary pressure data.

14. A medical system, **characterized in that**, comprising a ventilation device, an electrical impedance apparatus and a processor; wherein the ventilation device comprises a sensor and a breathing circuit, wherein the breathing circuit is configured to deliver a gas to a ventilation subject for mechanical ventilation; the sensor is configured to acquire ventilation pressure data that reflect a ventilation state, while providing the mechanical ventilation to the ventilation subject;
the electrical impedance apparatus comprises an unit for acquiring electrical impedance data and an unit for processing electrical impedance data; wherein the unit for acquiring electrical impedance data is configured to acquire electrical impedance signals, and the unit for processing electrical impedance data is configured to process the electrical impedance signals, so as to obtain the electrical impedance data;
the processor is configured to implement the data processing method based on electrical impedance according to any one of claims 1~13.

15. A ventilation device, **characterized in that**, comprising:
a breathing circuit, which is configured to deliver a gas to a ventilation subject for mechanical ventilation;
a sensor, which is configured to acquire ventilation pressure data that reflect a ventilation state, while providing the mechanical ventilation to the ventilation subject;
a communication module, which is in communicative connection with an electrical impedance apparatus, wherein the electrical impedance apparatus is configured to acquire electrical impedance data, while providing the mechanical ventilation to the ventilation subject; and
a processor, which is configured to implement the data processing method based on electrical impedance according to any one of claims 1~13.
